# EUROPÄISCHE PATENTSCHRIFT

(11) **EP 3 471 642 B1**
(45) Veröffentlichungstag und Bekanntmachung des Hinweises auf die Patenterteilung: **13.05.2020**
(21) Anmeldenummer: 17737208.3
(22) Anmeldetag: 30.05.2017
(51) Int. Cl.: A61B 18/14

(54) **CHIRURGISCHES INSTRUMENT FÜR DIE ELEKTROTOMIE UND WERKZEUG DAFÜR**
SURGICAL INSTRUMENT FOR ELECTROTOMY AND TOOL FOR SAME
INSTRUMENT CHIRURGICAL POUR LA DIATHERMIE CHIRURGICALE ET ACCESSOIRE POUR CELUI-CI

(30) Priorität: 15.06.2016 DE 102016007232
(43) Veröffentlichungstag der Anmeldung: 24.04.2019
(73) Patentinhaber: Reger Medizintechnik GmbH, 78667 Villingendorf (DE)
(72) Erfinder: HETZEL, Alexander, 78667 Villingendorf (DE)
(74) Vertreter: Mehl-Mikus, Claudia
(86) Internationale Anmeldenummer: PCT/EP2017/000630
(87) Internationale Veröffentlichungsnummer: WO 2017/215779

(56) Entgegenhaltungen:
- US-A1- 2006 235 377
- US-A1- 2015 105 777
- US-A1- 2015 157 773
- US-A1- 2015 238 245

## Beschreibung

Die Erfindung betrifft ein chirurgisches Instrument für die Elektrotomie und ein Werkzeug für ein solches chirurgisches Instrument.

Aus dem Stand der Technik sind chirurgische Instrumente für die Elektrotomie bekannt, die beispielsweise für endoskopische oder arthroskopische Operationsverfahren verwendet werden. Unter Elektrotomie ist das Schneiden oder Koagulieren von biologischem Gewebe zu verstehen, wobei mittels kleinflächiger Elektroden eine hohe Stromdichte an der Übertrittstelle des Stroms zum Gewebe erzeugt wird. Die dazu benötigte elektrische Leistung wird so gewählt, dass das Gewebe in kürzester Zeit auf über 60° bis über 100° Celsius erhitzt wird, die Zellstruktur des Gewebes ruptiert und die Zellflüssigkeit darin vaporisiert. Diese Technik wird entsprechend für arthroskopische Elektrochirurgie, nämlich für das Behandeln von Gewebe in Körperhöhlen oder Gelenken, vorgesehen und wird im Normalfall in Gegenwart eines Dehnmediums, das zum Aufblähen der zu behandelnden Körperstelle dient, oder eines salinen Fluids verwendet.

Die Elektrotomie wird auch Hochfrequenz (HF)-Chirurgie genannt, mit einem Wechselstrom in einer dafür vorgesehenen Frequenz von ca. 300 kHz bis 4 MHz betrieben und kann monopolar oder bipolar angewendet werden. Beim monopolaren Verfahren wird mit einer aktiven Elektrode am Werkzeug und einer neutralen oder auch Rückführ-Elektrode gearbeitet, die an einem Körperteil des Patienten angebracht ist. Das bipolare Verfahren unterscheidet sich vom monopolaren Verfahren, indem die neutrale Elektrode in unmittelbarer Nähe zu der aktiven Elektrode direkt am Werkzeug angeordnet sein kann. Der Weg, den der Strom durch den Körper nehmen muss, wird dadurch stark verkürzt. Hierfür werden unterschiedliche Elektroden bzw. Elektrodenanordnungen verwendet, die über unterschiedliche Steuerelemente aktiviert werden können.

Hierzu ist aus DE 10 2011 105 404 B4 ein chirurgisches Instrument für die Elektrotomie mit einem an einem Handgriff angeordneten stabförmigen Elektrodenhalter bekannt. Der Elektrodenhalter weist einen rohrförmigen Grundkörper auf, der aus einem elektrisch isolierenden Material beschaffen ist und eine pilzförmig ausgebildete aktive Elektrode trägt. Die aktive Elektrode ragt dabei durch eine Öffnung des distalen Endes des Grundkörpers hindurch. Elektrode sowie Grundkörper weisen einen innenliegenden Kanal auf, der zur Absaugung des mit der Elektrode behandelten Gewebes dient.

Die US 2015/238 245 A1 beschreibt ein Aktivelement, dessen proximales Ende in einen Tubus eines entsprechenden Werkzeugs oder Werkzeughalters gesteckt werden kann, so dass das Aktivelement als Elektrode genutzt werden kann. Die Form des Aktivelements ist länglich, wobei an der abgeflachten Spitze eine seitliche Öffnung vorgesehen ist, durch die Gewebe abgesaugt werden kann. Auf die Spitze kann ein Isolator gesteckt werden, der die Form der Spitze nachformt.

Aus der US 2006/235 377 A1 ist ein Instrument für die Elektrochirurgie bekannt, dessen Elektrode aus einem zylindrischen Lumen, das die Verbindung zum Instrument herstellt, und einem dazu senkrecht angeordneten zylindrischen Elektrodenstück aufgebaut ist. Beide zusammen sind modular, L-förmig zusammengesteckt. Ferner zeigt die US 2015/105 777 A1 eine Elektrode, die einen distalen Endabschnitt aufweist, der abgeknickt ausgebildet ist. Die US 2005/277 916 A1 offenbart ein Aktivelement, das in eine seitliche Öffnung eines rohrförmigen Instrumentenaufsatzes, wobei ein L-förmiger Absaugkanal gebildet wird.

Nachteilig beim vorgenannten Stand der Technik ist, dass in dem Grundkörper Endbereiche sowie an Übergangsabschnitten von Elektrode zu Absaugkanal Eckbereiche vorliegen, an und in denen sich Gewebe sammeln und schlecht aus dem Absaugkanal entfernt werden können. Der Absaugkanal kann verstopfen und das Werkzeug so nicht oder nur sehr schlecht sterilisiert werden und kann dadurch nicht weiter verwendet werden.

Ausgehend von diesem Stand der Technik ist es Aufgabe der vorliegenden Erfindung, ein verbessertes Werkzeug für ein chirurgisches Instrument für die Elektrotomie bereitzustellen, mit dem Gewebe während einer Operation leichter abgesaugt werden kann und das gut zu sterilisieren ist.

Diese Aufgabe wird durch ein Werkzeug mit den Merkmalen des Anspruchs 1 gelöst.

Die weitere Aufgabe, ein chirurgisches Instrument für die Elektrotomie bereitzustellen, das einfach zu verwenden ist und kostengünstig hergestellt werden kann, wird durch ein Instrument mit den Merkmalen des Anspruchs 10 gelöst.

Weiterbildungen bzw. bevorzugte Ausführungsformen des Werkzeugs sowie des chirurgischen Instruments sind in den Unteransprüchen ausgeführt.

Eine erste Ausführungsform eines erfindungsgemäßen Werkzeugs für ein chirurgisches Instrument für die Elektrotomie weist einen rohrförmigen Grundkörper auf, der an seinem distalen Ende eine Öffnung hat, die in der Seitenwandung des Grundkörpers (5) angeordnet ist, in der eine aktive, rohrförmige Elektrode angeordnet ist. Erfindungsgemäß hat die aktive Elektrode einen J-förmigen oder gebogen L-förmigen Elektrodenkörper, der einen kurzen, gebogenen ersten Abschnitt, der durch die Öffnung des Grundkörpers ragt, und einen länglichen, rohrförmigen zweiten Abschnitt aufweist, der sich in dem Grundkörper koaxial erstreckt. Dabei sind der erste Abschnitt und der zweite Abschnitt einstückig ausgebildet.

"J-förmig" oder auch "L-förmig" im Sinne der Erfindung meint einen Elektrodenkörper, der einen im Vergleich zu der Gesamtlänge des Elektrodenkörpers kurzen gebogenen Teil und einen sich darin anschließenden länglich ausstreckenden Schaft hat. Der gebogene Abschnitt weist dabei ausgehend von einer Längsachse dieses Schafts um 90 ° abgewinkelt von diesem Schaft zur Seite weg. Dadurch, dass der erste Abschnitt gebogen ist, also dort einen Radius aufweist, wo ansonsten Kanten waren, kann eine Ansammlung von Gewebe innerhalb des Werkzeugs nahezu vollkommen vermieden werden. Es liegen keine Ecken oder Kanten innerhalb des Absaugkanals vor, so dass Gewebe, das durch die Elektrode gelöst wird, ohne weiteres durch den Absaugkanal zügig abgesaugt werden kann.

Vorteilhaft an dieser Erfindung ist insbesondere, dass die Elektrode bzw. der Elektrodenkörper einstückig ist, d. h. aus einem einzigen, durchgängig geformten Bauteil aufgebaut ist, und aus Metall besteht. Dabei können neuste 3D-Drucktechnologien verwendet werden, sodass der Elektrodenkörper in einem Stück gefertigt werden kann. Es sind keine komplexen Fräs- oder Gusstechniken mehr notwendig, sondern der Elektrodenkörper kann mittels generativer Herstellungsmethoden, wie z. B. 3D-Druck produziert werden. Das Werkzeug kann hierdurch einfach aufgebaut werden und ist durch die gebogene Form der Elektrode auch einfach zu sterilisieren.

Der gebogene erste Abschnitt kann einen oder mehrere Biegungsradien haben, die den jeweiligen Anforderungen angepasst werden können. So kann ein innerer erster Biegungsradius des ersten Abschnitts in einem Bereich von 0,3 mm bis 3,0 mm und ein äußerer zweiter Biegungsradius kann in einem Bereich von 0,7 mm bis 5,0 mm liegen. Hierdurch kann eine Biegung des ersten Abschnitts so gewählt werden, dass eine möglichst leichte Absaugung erreicht werden kann.

In einer weiteren Ausführungsform der Erfindung ist vorgesehen, dass der erste Abschnitt der Elektrode ein flanschartiges Abschlusselement mit einem in Bezug zu der Öffnung des Grundkörpers radialen Überstand aufweisen kann, der an einem Außenrand der Öffnung des Grundkörpers anliegt. Das flanschartige Abschlusselement bildet ein flächiges Element, das außen an dem Grundkörper anliegt, die Öffnung vollständig überdeckt und zudem abdichtet. Hierdurch wird an der Außenseite des Grundkörpers eine Elektrodenfläche gebildet, die eine beliebig zu wählende Form und Abmessung aufweisen kann. So können verschiedene Elektrodenformen realisiert werden, wobei das Abschlusselement kreisförmig, oval bzw. auch rechteckig geformt sein kann.

Ferner kann das flanschartige Abschlusselement eine Abschlussplatte mit Absaugöffnungen sein. Durch diese Absaugöffnungen kann Gewebe einfach in den dahinterliegenden Absaugkanal gelangen. Diese Absaugöffnungen können unterschiedliche Abmessungen haben, wobei eine einzige Absaugöffnung oder auch mehrere kleinere Absaugöffnungen realisiert werden können. Wird nur eine einzige Absaugöffnung realisiert, kann eine vergleichsweise große Öffnung ausgebildet werden, die größere Gewebestücke aufnehmen kann, und für Arbeiten an großen Gelenken in Frage kommt, wie bspw. für eine Hüfte. Hat die Abschlussplatte mehrere kleinere Absaugöffnungen, können diese nicht so schnell verstopfen und sind für Arbeiten an kleineren Gelenken, in denen die gelösten Gewebeteile in der Regel kleiner sind, wie bspw. für den Schulterbereich, gut geeignet.

In einer weiteren Ausführungsform der Erfindung kann vorgesehen sein, dass der rohrförmige Grundkörper mehrteilig ist. Dabei kann der Grundkörper ein Distalbauteil und ein Proximalbauteil aufweisen, wobei ein Außendurchmesser des Proximalbauteils sowie ein Innendurchmesser des Distalbauteils so gewählt sein können, dass das Proximalbauteil lösbar koaxial in dem Distalbauteil aufgenommen ist. Ein Überlappbereich des Distalbauteils und des Proximalbauteils bildet einen Befestigungsabschnitt für beide Bauteile, so dass ein zusammengesetzter Grundkörper gebildet wird.

Beide Bauteile sind im Wesentlichen rohrförmig und weisen einen Innenkanal auf, der zur Gewebeabsaugung dient. Das Distalbauteil hat eine geschlossene Stirnseite und weist zur Aufnahme des Elektrodenkörpers die Öffnung des Grundkörpers seitlich angrenzend in der Seitenwandung des Distalbauteils auf. Hiermit lässt sich Gewebe aus Gelenkpfannen einfacher erreichen und gezielter entfernen als mit den aus dem Stand der Technik bekannten Werkzeugen. Das Distalbauteil kann einen inneren Kanal haben, der in Form einer Sackbohrung geformt ist. Alternativ kann der Kanal auch der Form des Elektrodenkörpers folgen und eine Biegung aufweisen, die zu der Biegung des gebogenen ersten Abschnitts korrespondiert und den Abschnitt damit stützt.

Der Überlapp der beiden Bauteile kann so gestaltet sein, dass ein distales Ende des Proximalbauteils innen an der Stirnseite des Distalbauteils teilweise anliegen kann. Damit eine Verbindung des Innenkanals des Proximalkanals und der Öffnung entsteht, so dass die Elektrode eingesetzt werden kann, ist ein distales Ende des Proximalkanals angeschrägt bzw. weist eine entsprechende Ausnehmung auf. Ein distaler Abschnitt des Proximalbauteils ist dabei so ausgebildet, dass der Elektrodenkörper einfach eingesteckt werden kann. Da eine Wandung des Proximalbauteils länger ausgebildet sein kann, als eine gegenüberliegende Wandung, kann der Elektrodenkörper einfach eingeschoben werden und bleibt in dieser Position auch gehalten.

Ferner kann erfindungsgemäß vorgesehen sein, dass an seinem den Befestigungsabschnitt bildenden Ende das Proximalbauteil über eine Stufe eine Außenumfangsverjüngung aufweist. Das Distalbauteil kann an dem verjüngten Außenumfang anliegen, wobei ein zu der Stufe des Proximalbauteils weisendes Ende des Distalbauteils von der Stufe beabstandet ist. Die Stufe dient dazu, einen Befestigungsabschnitt zu bilden, in dem das Proximalbauteil gecrimpt bzw. angeschweißt werden kann. Diese Befestigungsart ist einfach und erzeugt eine sichere und feste Verbindung der Bauteile.

Dieser Abstand bewirkt, dass sich eine Befestigungseinrichtung bildet, so dass die beiden Bauteile in ihrer ineinandergesteckten Form sich nicht voneinander lösen können.

Dazu kann in einer noch weiteren Ausführungsform die Erfindung insbesondere vorsehen, dass eine Isolierschicht um das Proximalbauteil und zumindest um einen Teil des Befestigungsabschnitts anliegt. Dabei kann die Isolierschicht eine hülsenförmigen Isolierung, ein Isolationsrohr aus Kunststoff oder auch ein Schrumpfschlauch sein. Hierbei bilden die freiliegende Außenumfangsverjüngung des Proximalbauteils und ein Ende des Distalbauteils eine Anlagefläche für die Isolierschicht. Diese Isolierschicht, bspw. der Schrumpfschlauch, kann so an beiden Grundkörperbauteilen, nämlich dem Distalbauteil und dem Proximalbauteil, anliegen, dass beide von der Isolierschicht überlappend abgedeckt werden. Das Proximalbauteil kann in proximaler Erstreckung auch vollständig mit der Isolierschicht bedeckt sein.

Die Erfindung kann auch vorsehen, dass das Distalbauteil an seinem Ende, an dem der Befestigungsabschnitt vorliegt, über eine Stufe eine Außenumfangsverjüngung aufweist, und das Proximalbauteil über eine Stufe eine Innenumfangsverbreiterung zeigt, wobei das Proximalbauteil an dem verjüngten Außenumfang des Distalbauteils zur Anlage kommt. Dabei liegt die Stufe des Distalbauteils an dem distalen Ende des Proximalbauteils und die Stufe des Proximalbauteils liegt an dem proximalen Ende des Distalbauteils an, so dass sich beide Bauteile korrespondierend überlappen. Die Umfangsverjüngung/- verbreiterung liegen dabei vollumfänglich aneinander an. Eine Isolierung kann über den gesamten Außenumfang bzw. Mantelteil des Proximalbauteils vorliegen und sich entlang dessen gesamter Länge erstrecken. Im Bedarfsfall, falls das Proximalbauteil nicht aus Metall, sondern aus Kunststoff besteht, kann die Isolierung auch ganz entfallen.

Alternativ kann die Erfindung vorsehen, dass die Bauteile des Grundkörpers zueinander korrespondierende Rastelemente aufweisen, so dass sie durch Einrasten in dem Befestigungsabschnitt miteinander verbunden werden können. Die Bauteile können aber, sobald sie montiert, d.h. ineinander eingesteckt, sind, miteinander verquetscht, gecrimpt oder auch verschweißt oder verklebt werden, um sicher in der Montageanordnung zu verbleiben.

Durch die erfindungsgemäßen Befestigungsmöglichkeiten lassen sich beide Bauteile des Grundkörpers so miteinander verbinden, dass das Distalbauteil sicher gehalten ist und das Werkzeug sicher isoliert ist.

Die Erfindung kann ferner vorsehen, dass der Elektrodenkörper sich zumindest so weit in den Grundkörper erstreckt, dass er - in proximaler Richtung des Werkzeugs erstreckend - bis zu der Stufe des Proximalbauteils, bevorzugt weiter als bis zu einer inneren Abstufung bzw. einer Ringstufe reichen kann. Der Elektrodenkörper kann teilweise in dem Proximalbauteil aufgenommen sein, oder sich in dessen Innerem über seine gesamte Länge erstrecken. Die beiden wesentlichen Abschnitte des Elektrodenkörpers können unterschiedlich lang sein. Der gebogene erste Abschnitt kann im Vergleich zu dem länglichen zweiten Abschnitt kurz sein und der zweite Abschnitt im Vergleich dazu eine vielfache Länge haben und weit in den Proximalbauteil hineinreichen. Der zweite Abschnitt kann aber auch in der Länge variieren. Für eine gute Absaugung und gute Halterung erstreckt sich der zweite Abschnitt über den Befestigungsabschnitt des Grundkörpers hinaus, weit in den Innenkanal des Proximalbauteils bzw. bis an dessen proximales Ende. Allerdings kann der zweite Abschnitt aber auch sehr kurz sein, wobei er die gleichen oder nur geringfügig längere Abmessungen als der erste Abschnitt haben kann. In einer weiteren Ausführungsform verjüngt sich der Elektrodenkörper bzw. dessen Innenkanal in distaler Richtung, so dass sich der Kanal in proximaler Richtung erweitert. Dies begünstigt eine gute Absaugung, so dass, falls sich im distalen Bereich Gewebe verklemmt oder fest sitzt, die Verstopfung sich einfach lösen kann.

Damit eine gute Kontaktierung des Elektrodenkörpers mit dem Proximalbauteil erreicht werden kann, sieht die Erfindung ferner vor, dass der Elektrodenkörper an seinem proximalen Ende einen verdickten Außendurchmesser aufweist, dessen Mantelfläche an der Innenseite des Proximalbauteils anliegt. Hierdurch wird ein Schleifkontakt gebildet, der eine Stromversorgung des Elektrodenkörpers und damit die Nutzung als aktive Elektrode ermöglicht. Die aktive Elektrode kann aber auch direkt kontaktiert werden - hierzu kann der Elektrodenkörper sich koaxial bis zu einem proximalen Ende des Werkzeugs erstrecken und dort in geeigneter Weise kontaktiert werden. Ferner können auch andere Kontaktierungsmethoden verwendet werden, so ist neben einer Schleifkontaktierung auch die Verwendung von Bandleitern, Steckkontakten, Federkontakten usw. möglich.

Die Erfindung sieht ferner vor, dass das Werkzeug ein bipolares Werkzeug ist und eine neutrale Elektrode aufweisen kann, die hülsenförmig koaxial zu dem Proximalbauteils des Grundkörpers angeordnet sein kann. Die neutrale Elektrode kann, muss aber nicht unbedingt bis zum Ende des Proximalbauteils ausgebildet sein. Sie kann sich auch in distaler Richtung entlang des Grundkörpers bis zu einem bestimmten Punkt erstrecken, sodass zwischen der neutralen Elektrode und der aktiven Elektrode ein gewisser Abstand besteht. Hierdurch wird die Gefahr von Kurzschlüssen verringert.

Damit das Werkzeug bei Verwendung einer neutralen Elektrode nicht zu Kurzschlüssen bzw. Verletzungen von gesundem Gewebe führt, kann über die neutrale Elektrode eine weitere Isolierung in Form einer Schicht, Beschichtung, einem aufgeschrumpften Kunststoffschlauch oder einer aufgeschobenen Kunststoffhülse angebracht sein.

Um das Werkzeug in einem Instrument zur Elektrotomie nutzen zu können, sieht die Erfindung vor, dass die aktive Elektrode, und damit der Elektrodenkörper aus Metall, bevorzugt einem Edelstahl, besonders bevorzugt einem medizinischen Edelstahl gefertigt sind. Zur Herstellung des Elektrodenkörpers kann ein 3D-Druckverfahren verwendet werden, wobei die Form beliebig an ein individuell anzufertigendes Werkzeug oder spezielle Elektrodenformen angepasst werden kann. Es können auch andere generative Herstellungsverfahren genutzt werden. Ferner kann das Distalbauteil des Grundkörpers aus einem elektrisch isolierenden Material, bevorzugt einem Kunststoff oder einer Keramik und das Proximalbauteil des Grundkörpers aus Metall bestehen. Hierdurch kann erreicht werden, dass der Elektrodenkörper gut mit Strom versorgt wird und dass er nach außen isoliert wird. Alternativ kann auch vorgesehen sein, dass das Proximalbauteil ebenfalls aus Kunststoff besteht; auf eine separate Isolierung kann dann verzichtet werden. In diesem Fall kann die Elektrode an ihrem proximalen Ende mit den im Handgriff vorliegenden elektrischen Kontakten kontaktiert werden, wozu Schleifkontakte oder Steckkontakte vorgesehen sein können.

Ferner kann vorgesehen sein, dass die neutrale Elektrode wiederum von einer hülsenförmigen Isolierschicht bzw. einem Isolationsrohr umgeben ist. Somit wird eine Elektrodenanordnung aus aktiver und neutraler Elektrode nur an einem distalen Ende des Werkzeugs erreicht. In einer beispielhaften Abmessung hat ein Werkzeug eine Länge von 100 bis 300 mm, wobei im Vergleich dazu der distale Endabschnitt eine Länge von 10 bis 100 mm hat und davon die neutrale Elektrode einen Abschnitt von 5 bis 10 mm übernimmt. Hierdurch wird ein besonders handliches Werkzeug geschaffen.

Ein chirurgisches Instrument für die Elektrotomie weist einen Handgriff und ein mit dem Handgriff lösbar verbindbares Werkzeug auf, wobei das Werkzeug ein erfindungsgemäßes Werkzeug ist.

Damit wird ein besonders einfaches Instrument erhalten, das eine sichere und schnelle Absaugung von Gewebe ermöglicht.

In einer Weiterbildung der Erfindung ist vorgesehen, dass der Handgriff an seinem proximalen Ende eine Anschlusseinrichtung aufweist, wobei die Anschlusseinrichtung eine elektrische Verbindung der Elektrode zu einer Stromversorgung bzw. eine schlauchförmige Verbindung zu einer Absaugvorrichtung ist. Der Handgriff ist insbesondere ergonomisch ausgebildet und weist verschiedene Bedienelemente auf, womit bspw. die Elektrode unter Strom gesetzt werden und das Werkzeug einfach ausgewechselt werden kann. Ein passender Handgriff ist u.a. in der DE 10 2011 016 585 beschrieben. Zur Aufnahme und Halterung des Werkzeugs sind verschiedenste Konstruktionsmöglichkeiten bekannt.

Das Werkzeug kann in einer spezifischen Weise montiert bzw. hergestellt werden. Zunächst werden die Bauteile durch bekannte Herstellungsverfahren, wie Spritzguss, Frästechnik oder auch 3D-Druck angefertigt. Insbesondere der Elektrodenkörper lässt sich mittels einer 3D-Drucktechnik oder mittels einer anderen generativen Fertigungsweise direkt einstückig fertigen. Nach Bereitstellen der Bauteile werden diese in einer bestimmten Reihenfolge zusammengesetzt. Zunächst wird der Elektrodenkörper in das Distalbauteil eingesetzt. Dadurch, dass dieses aus Kunststoff ist und das Anschlusselement plan anliegt, muss die Öffnung nicht separat abgedichtet werden. Hiernach kann das Proximalbauteil in das Distalbauteil eingesetzt werden, wobei der vordere, verjüngte Abschnitt des Proximalbauteils zwischen der Wandung des Distalbauteils und der Wandung des Elektrodenkörpers geführt wird. Kommt die Stirnseite des Proximalbauteils an der inneren Stirnseite des Distalbauteils zu liegen, wird über das Proximalbauteil und einen Bereich des Distalbauteils von proximaler Richtung die Isolierung aufgebracht, so bspw. ein Isolierrohr aufgezogen oder ein Schrumpfschlauch aufgezogen und aufgeschrumpft. Bei Verwendung einer neutralen Elektrode wird dieser aufgesteckt und schließlich eine abschließende Isolierung von proximaler Seite aufgezogen. Das Werkzeug ist in dieser Form dann einsatzbereit.

Durch die Herstellung mittels 3D-Druck ergeben sich technische Vorteile, insbesondere wenn der aktive Kopf gedruckt wird. Auch bereits bestehende Systeme und Handgriffe können mit dem erfindungsgemäßen Werkzeug nachgerüstet oder einfach verwendet werden. Auch durch seinen einfachen Aufbau trägt das Werkzeug dazu bei, ein einfach zu verwendendes Instrument bereitzustellen.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine Seitenansicht eines erfindungsgemäßen chirurgischen Instruments,
- **Fig. 2**: eine weitere zur Fig. 1 gedrehte Seitenansicht des chirurgischen Instruments,
- **Fig. 3**: eine Schnittdarstellung durch einen distalen Endabschnitt A-A einer Ausführungsform eines erfindungsgemäßen Werkzeugs, und
- **Fig. 4**: eine Schnittdarstellung durch einen distalen Endabschnitt einer weiteren Ausführungsform des Werkzeugs.

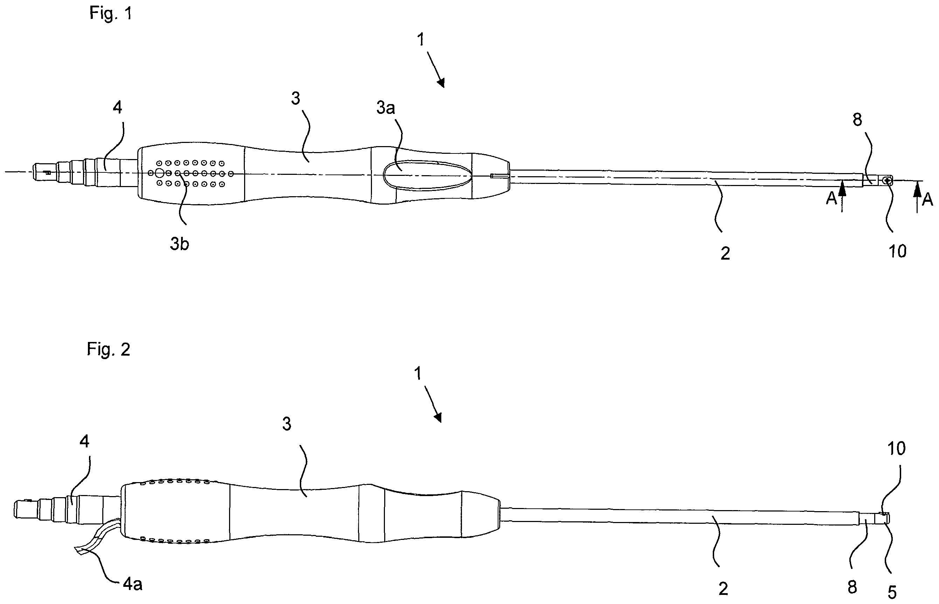

Ein chirurgisches Instrument 1 für die Elektrotomie weist, wie **Fig. 1** und **2** zeigen, ein Werkzeug 2 auf, das auf einen Handgriff 3 lösbar aufgesteckt werden kann. Der Handgriff 3 ist mit einem Bedienelement 3a und Noppen 3b für rutschfestes Halten des Griffs 3 versehen. An seinem proximalen Ende weist der Handgriff 3 eine Anschlusseinrichtung 4 auf, die dazu dient, einen Schlauch mit einer Absaugvorrichtung (nicht figurativ dargestellt) zu verbinden. Ferner ist ein elektrischer Anschluss 4a an der Anschlusseinrichtung 4 vorgesehen, die das Werkzeug 2 elektrisch mit einer Stromversorgung verbindet.

Das Werkzeug 2 ist stabförmig und weist an seinem distalen Endbereich eine Elektrode 10 auf, die seitlich an dem Werkzeug 2 an einem Grundkörper 5 angeordnet ist. Um den runden Grundkörper 5 ist koaxial eine weitere hülsenförmige Elektrode 8 angeordnet. Die erstgenannte Elektrode 10 wird als aktive Elektrode verwendet, wohingegen die hülsenförmige Elektrode 7 als neutrale Elektrode genutzt wird.

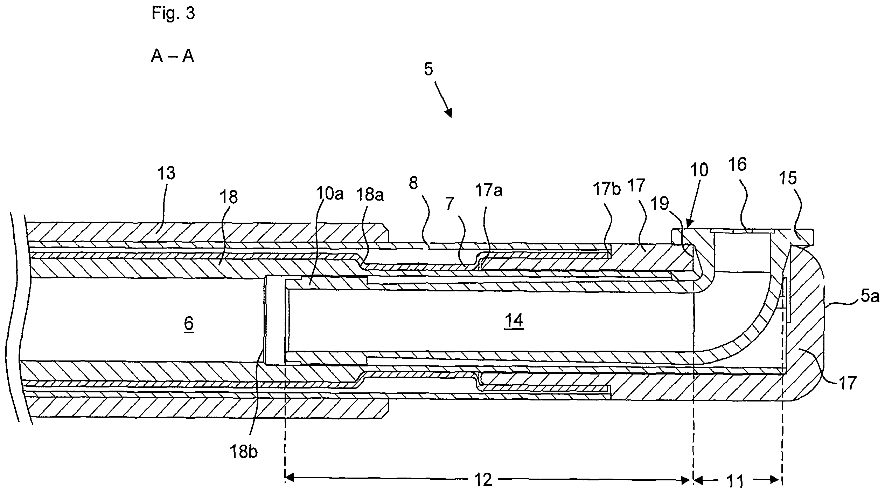

Der distale Endbereich ist gemäß Schnitt A-A in **Fig. 1** näher in **Fig. 3** gezeigt, wobei das Werkzeug 2 im Wesentlichen einen rohrförmigen Grundkörper 5 hat. Der Grundkörper 5 ist mehrteilig, wobei er aus einem Distalbauteil 17 und einem Proximalbauteil 18 aufgebaut ist. Das Distalbauteil 17 besteht aus einem Kunststoff, umschließt die Elektrode 10 und wirkt gleichzeitig als Isolierung für die Elektrode 10. Das Proximalbauteil 18 besteht aus Metall.

Das Proximalbauteil 18 ist röhrenförmig und weist einen Absaugkanal 6 und über eine Stufe 18a eine Außenumfangsverjüngung auf. Das Distalbauteil 17 ist so auf das distale Ende des Proximalbauteils 18 aufgesteckt, so dass es an dem verjüngten Außenumfang des Proximalbauteils 18 anliegt. Dabei ist ein zu der Stufe 18a weisendes Ende des Distalbauteils 17 von der Stufe 18a beabstandet. Ein Außendurchmesser des Proximalbauteils 18 und ein Innendurchmesser des Distalbauteils 17 sind so gewählt, dass das Proximalbauteil 18 lösbar koaxial in dem Distalbauteil 17 aufgenommen ist und sich beide Bauteile in einem Befestigungsabschnitt überlappen.

Um das Proximalbauteil 18 und einen Teil des Befestigungsabschnitts liegt eine Isolierung 7 an. Die Isolierung kann ein Schrumpfschlauch sein, der einfach bei Montage des Werkzeugs aufgeschrumpft wird. Die Außenumfangsverjüngung des Proximalbauteils 18 und ein Ende 17a des Distalbauteils 17 bilden dabei eine Anlagefläche für die Isolierung 7.

Die Isolierung 7 erstreckt sich in distaler Richtung bis zu einer Stufe 17b des Distalbauteils 17, wozu das Distalbauteil 17 eine Umfangsverjüngung an seinem proximalen Endbereich aufweist.

Die Elektrode 10, die in dem Distalbauteil angeordnet ist, hat einen Elektrodenkörper, der im Wesentlichen in dem Distalbauteil 17 aufgenommen ist. Das Distalbauteil 17 weist dazu eine Öffnung 9 auf, die nicht an einer Stirnseite 5a des Grundkörpers 5, sondern an einer Seitenwandung angeordnet ist. Hiermit lässt sich Gewebe aus Gelenkpfannen einfacher erreichen und gezielter entfernen.

Der Elektrodenkörper ist einstückig rohrförmig ausgebildet und kann sehr einfach durch 3D-Druckverfahren hergestellt werden. Er ist im Wesentlichen J-förmig und hat zwei Abschnitte: Einen kurzen, gebogenen ersten Abschnitt 11, der durch die 9 des Grundkörpers 5 ragt, und einen länglichen, rohrförmigen zweiten Abschnitt 12, der sich in dem Grundkörper 5 koaxial erstreckt. Die beiden Abschnitte gehen dabei ineinander über, weil der Elektrodenkörper einstückig, also aus einem Stück bestehend, ausgebildet ist. Da der Elektrodenkörper rohrförmig ist, ist im Inneren der Elektrode 10 ein Kanal 14 gebildet, der zu dem Absaugkanal 6 verbunden ist.

Der Elektrodenkörper erstreckt sich in einer bestimmten Weise in den Grundkörper 5, wobei er bis hinter die Stufe 18a des Proximalbauteils 18 reicht. Die Elektrode 10 weist an einem proximalen Ende 10a des Elektrodenkörpers einen verdickten Außendurchmesser auf, dessen Mantelfläche an der Innenseite des Proximalbauteils 18 anliegt. Damit wird die elektrische Verbindung zu dem Proximalbauteil 18 und damit der Stromversorgung hergestellt. Damit der Elektrodenkörper nicht zu weit in den Absaugkanal 6 rutscht, ist an der Außenseite des Kanals 6 eine Ringstufe 18b ausgeformt, die aber nicht inkrementell, sondern konisch geformt ist. Die Ringstufe 18b ist an dem Außenbereich der aktiven Elektrode 10 ausgebildet. Diese Ringstufe 18b kann einfach an dem betreffenden Bereich dargestellt werden.

Der erste Abschnitt 11 der Elektrode 10, der ein flanschartiges Abschlusselement mit einem in Bezug zu der Öffnung 9 des Grundkörpers 5 radialen Überstand 15 hat, der an einem Außenrand der Öffnung 9 des Grundkörpers 5 anliegt. Die Anlage an den Kunststoff des Distalbauteils 17 wirkt wie eine Dichtung zwischen Abschlusselement und dem Grundkörper 5.

Das flanschartige Abschlusselement ist im Wesentlichen eine Abschlussplatte, die Absaugöffnungen 16 aufweist. Diese Absaugöffnungen sind direkt mit dem Kanal 14 und über diesen mit dem Absaugkanal 6 des Werkzeugs 2 verbunden. Gewebe, das durch Verwendung der Elektrode 10 gelöst wird, kann direkt durch die Elektrode 10 abgesaugt werden, wobei das Gewebe im Kanal 14 einfach durch den gebogenen Abschnitt gesaugt wird und an keiner Kante oder Ecke hängenbleiben kann.

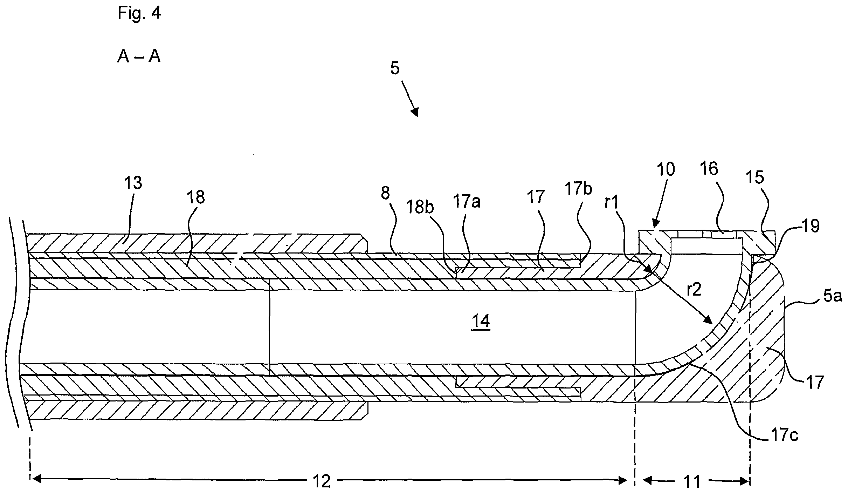

In **Fig. 4** zeigt eine weitere Bauform des Werkzeugs 2, eine zu **Fig. 3** veränderte Montageanordnung der einzelnen Bauteile.

Das Distalbauteil 17 zeigt an seinem Ende, an dem der Befestigungsabschnitt vorliegt, über eine Stufe 17b eine Außenumfangsverjüngung. Korrespondierend dazu hat das Proximalbauteil 18 über Stufe 18b eine Innenumfangsverbreiterung, wobei das Proximalbauteil 18 an dem verjüngten Außenumfang des Distalbauteils 17 anliegt. Dabei kommt die Stufe 17b des Distalbauteils 17 an dem distalen Ende des Proximalbauteils 18 und die Stufe 18b des Proximalbauteils 18 an dem proximalen Ende 17a des Distalbauteils 17 zur Anlage, so dass sich beide Bauteile korrespondierend überlappen.

Das Proximalbauteil 18 besteht in der Variante der **Fig. 4** nicht aus Metall, sondern einem Kunststoff. Eine separate Isolierung ist daher nicht notwendig. Die Kontaktierung der Elektrode 10 erfolgt an deren proximalen Ende. Dazu erstreckt sich der zweite Abschnitt 12 der Elektrode 10 bis zu einem elektrischen Anschluss der mit der elektrischen Anschlusseinrichtung 4 des Handgriffs 3 in einer Montageanordnung in Eingriff steht. Es kann ein Schleifkontakt, ein Federkontakt oder auch andere geeignete Kontakte verwendet werden. Die neutrale Elektrode 8 liegt als Hülse um die gesamte Länge des Proximalbauteils 18 herum an. Um die neutrale Elektrode 8 ringförmig auszubilden und den Schaft des Werkzeugs 2 weiter zu isolieren, ist ein Isolierrohr 13 koaxial zu der neutralen Elektrode 8 angeordnet.

Ferner ist der gebogene, erste Abschnitt 11 der Elektrode 10 mit verschiedenen Biegungsradien versehen. Ein innerer Radius r1 des ersten Abschnitts 11 liegt zwischen 0,3 mm und 3 mm, ein äußerer Radius r2 des ersten Abschnitts 11 liegt zwischen 0,7 mm und 5 mm. Das Distalbauteil 17 ist in den Bereich, in dem der erste Abschnitt 11 der Elektrode 10 gehalten ist, so ausgeformt, dass der gebogene erste Abschnitt 11 komplett umschlossen wird. Der innere Kanal des Distalbauteils 17 entspricht in der Form und Abmessung der Form und Abmessung des Mantels dem Elektrodenkörpers der Elektrode 10. Es entsteht damit eine gebogene Innenfläche 17c, die genau die Biegung des im ersten Abschnitts 11 der Elektrode 10 abbildet.

## Patentansprüche

1. Werkzeug (2),für ein chirurgisches Instrument für die Elektrotomie,
mit einem rohrförmigen Grundkörper (5), der an seinem distalen Ende eine Öffnung (9) aufweist, die in der Seitenwandung des Grundkörpers (5) angeordnet ist und in der eine aktive rohrförmige Elektrode (10) angeordnet ist,
wobei die aktive Elektrode (10) einen J-förmigen Elektrodenkörper hat, der einen kurzen, gebogenen ersten Abschnitt (11), der durch die Öffnung (9) des Grundkörpers (5) ragt, und einen länglichen, rohrförmigen zweiten Abschnitt (12) aufweist, der sich in dem Grundkörper (5) koaxial erstreckt, und wobei der erste Abschnitt und der zweite Abschnitt (12) einstückig ausgebildet sind.

2. Werkzeug (2) nach Anspruch 1,
**dadurch gekennzeichnet, dass**
der erste Abschnitt (11) der Elektrode (10) ein flanschartiges Abschlusselement mit einem in Bezug zu der Öffnung (9) des Grundkörpers (5) radialen Überstand (15) aufweist, der an einem Außenrand der Öffnung (9) des Grundkörpers (5) anliegt.

3. Werkzeug (2) nach Anspruch 2,
**dadurch gekennzeichnet, dass**
das flanschartige Abschlusselement eine Abschlussplatte ist, die Absaugöffnungen (16) aufweist.

4. Werkzeug (2) nach zumindest einem der Ansprüche 1 bis 3,
**dadurch gekennzeichnet, dass**
der rohrförmige Grundkörper (5) mehrteilig ist und ein Distalbauteil (17) und ein Proximalbauteil (18) aufweist, wobei ein Außendurchmesser des Proximalbauteils (18) und ein Innendurchmesser des Distalbauteils (17) so gewählt sind, dass das Proximalbauteil (18) lösbar koaxial in dem Distalbauteil (17) aufgenommen ist, wobei ein Überlappbereich von Distalbauteil (17) und Proximalbauteil (18) einen Befestigungsabschnitt bildet.

5. Werkzeug (2) nach Anspruch 4,
**dadurch gekennzeichnet, dass**
an seinem den Befestigungsabschnitt bildenden Ende das Proximalbauteil (18) über eine Stufe (18a) eine Außenumfangsverjüngung aufweist, und das Distalbauteil (17) an dem verjüngten Außenumfang zur Anlage kommt, wobei ein zu der Stufe (18a) des Proximalbauteils (18) weisendes Ende des Distalbauteils (17) von der Stufe (18a) beabstandet ist.

6. Werkzeug (2) nach einem der Ansprüche 1 bis 3,
**dadurch gekennzeichnet, dass**
das Distalbauteil (17) an seinem den Befestigungsabschnitt bildenden Ende über eine Stufe (17b) eine Außenumfangsverjüngung aufweist und das Proximalbauteil (18) korrespondierend über eine Stufe (18b) eine Innenumfangsverbreiterung aufweist, wobei das Proximalbauteil (18) an dem verjüngten Außenumfang des Distalbauteils (17) anliegt.

7. Werkzeug (2) nach Anspruch 5,
**dadurch gekennzeichnet, dass**
eine Isolierschicht, bevorzugt eine hülsenförmige Isolierung (7), besonders bevorzugt ein Schrumpfschlauch, um das Proximalbauteil (18) und zumindest um einen Teil des Befestigungsabschnitts anliegt,
wobei die Außenumfangsverjüngung des Proximalbauteils (18) und/oder ein Ende des Distalbauteils (17) eine Anlagefläche für die Isolierschicht bilden.

8. Werkzeug (2) nach zumindest einem der Ansprüche 4 bis 7,
**dadurch gekennzeichnet, dass**
der Elektrodenkörper
- sich zumindest so weit in den Grundkörper (5) erstreckt, dass er bis zu der Stufe (18a) des Proximalbauteils (18), bevorzugt weiter als bis zu der Stufe (18a) des Proximalbauteils (18) reicht, und/oder
- an seinem proximalen Ende (10a) einen verdickten Außendurchmesser aufweist, dessen Mantelfläche an der Innenseite des Proximalbauteils (18) anliegt.

9. Werkzeug (2) nach zumindest einem der Ansprüche 4 bis 8,
**dadurch gekennzeichnet, dass**
das Werkzeug (2) ein bipolares Werkzeug (2) ist und eine neutrale Elektrode (8) aufweist, die hülsenförmig koaxial zu dem Proximalbauteil (18) des Grundkörpers (5) angeordnet ist.

10. Chirurgisches Instrument (1) für die Elektrotomie mit einem Handgriff (3) und einem mit dem Handgriff (3) lösbar verbindbaren Werkzeug,
**dadurch gekennzeichnet, dass**
das Werkzeug ein Werkzeug (2) nach zumindest einem der Ansprüche 1 bis 9 ist.

11. Instrument (1) nach Anspruch 10,
**dadurch gekennzeichnet, dass**
der Handgriff (3) an seinem proximalen Ende eine Anschlusseinrichtung (4, 4a) aufweist, wobei die Anschlusseinrichtung (4, 4a) eine elektrische Verbindung der Elektrode (10) zu einer Stromversorgung und/oder eine schlauchförmige Verbindung zu einer Absaugvorrichtung ist.

## Claims

1. A tool (2) for a surgical instrument for electrotomy,
having a tubular main body (5) which has at its distal end an opening (9) which is arranged in a side wall of the main body (5) and in which an active electrode (10) is arranged,
**wherein**
the active electrode (10) has a J-shaped electrode body having a short, bent first portion (11), which protrudes through the opening (9) of the main body (5), and an elongate, tubular second portion (12), which extends coaxially in the main body (5), and wherein the first portion and the second portion (12) have an integral embodiment.

2. The tool (2) as claimed in claim 1,
**characterized in that**
the first portion (11) of the electrode (10) has a flange-like termination element with a protrusion (15) that is radial in relation to the opening (9) of the main body (5), said protrusion resting against an outer edge of the opening (9) of the main body (5).

3. The tool (2) as claimed in claim 2,
**characterized in that**
the flange-like termination element is a termination plate that has suction openings (16).

4. The tool (2) as claimed in at least one of claims 1 to 3,
**characterized in that**
the tubular main body (5) is of multipart design and has a distal component (17) and a proximal component (18), wherein an external diameter of the proximal component (18) and an internal diameter of the distal component (17) are selected such that the proximal component (18) is received in a detachable coaxial manner in the distal component (17), wherein an overlap region of the distal component (17) and proximal component (18) forms a fastening portion.

5. The tool (2) as claimed in claim 4,
**characterized in that**
the proximal component (18), at its end forming the fastening portion, has an external circumference taper by way of a step (18a) and the distal component (17) comes to rest against the tapered external circumference, wherein
an end of the distal component (17) pointing to the step (18a) of the proximal component (18) is spaced apart from the step (18a).

6. The tool (2) as claimed in any one of claims 1 to 3,
**characterized in that**
the distal component (17), at its end forming the fastening portion, has an external circumference taper by way of a step (17b) and the proximal component (18) correspondingly has an internal circumference broadening by way of a step (18b), wherein the proximal component (18) rests against the tapered external circumference of the distal component (17).

7. The tool (2) as claimed in claim 5,
**characterized in that**
an insulating layer, preferably a sleeve-shaped insulation (7), particularly preferably shrink tubing, rests around the proximal component (18) and at least around part of the fastening portion,
wherein the external circumference taper of the proximal component (18) and/or an end of the distal component (17) form an abutment face for the insulating layer.

8. The tool (2) as claimed in at least one of claims 4 to 7,
**characterized in that**
the electrode body
- extends at least so far into the main body (5) that it reaches up to the step (18a) of the proximal component (18), preferably reaches further than the step (18a) of the proximal component (18), and/or
- has a thickened external diameter at its proximal end (10a), the lateral face of which rests against the inner side of the proximal component (18).

9. The tool (2) as claimed in at least one of claims 4 to 8,
**characterized in that**
the tool (2) is a bipolar tool (2) and has a neutral electrode (8), which is arranged in a sleeve-shaped coaxial manner in relation to the proximal component (18) of the main body (5).

10. A surgical instrument (1) for electrotomy having a handle (3) and a tool that is detachably connectable to the handle (3),
**characterized in that**
the tool is a tool (2) as claimed in at least one of claims 1 to 9.

11. The instrument (1) as claimed in claim 10,
**characterized in that**
the handle (3) has at its proximal end a connection device (4, 4a), wherein the connection device (4, 4a) is an electrical connection of the electrode (10) to a power supply and/or a tube-shaped connection to a suction apparatus.

## Revendications

1. Outil (2) pour un instrument chirurgical pour l'incision à l'électrocautère, avec un corps de base tubulaire (5) qui présente à son extrémité distale une ouverture (9) qui est disposée dans la paroi latérale du corps de base (5) et dans laquelle une électrode tubulaire active (10) est disposée,
**dans lequel**
l'électrode active (10) a un corps d'électrode en forme de J qui présente une première section courbée courte (11) qui se dresse à travers l'ouverture (9) du corps de base (5) et une seconde section tubulaire oblongue (12) qui s'étend coaxialement dans le corps de base (5), et dans lequel la première section et la seconde section (12) sont réalisées d'une pièce.

2. Outil (2) selon la revendication 1,
**caractérisé en ce que**
la première section (11) de l'électrode (10) présente un élément d'arrêt de type bride avec une saillie radiale (15) par rapport à l'ouverture (9) du corps de base (5) qui repose sur un bord externe de l'ouverture (9) du corps de base (5).

3. Outil (2) selon la revendication 2,
**caractérisé en ce que**
l'élément d'arrêt de type bride est une plaque d'arrêt qui présente des ouvertures d'aspiration (16).

4. Outil (2) selon au moins une des revendications 1 à 3,
**caractérisé en ce que**
le corps de base tubulaire (5) est en plusieurs parties et présente un composant distal (17) et un composant proximal (18), dans lequel un diamètre externe du composant proximal (18) et un diamètre interne du composant distal (17) sont choisis de sorte que le composant proximal (18) est reçu de manière amovible coaxialement dans le composant distal (17), dans lequel une région de chevauchement du composant distal (17) et composant proximal (18) forme une section de fixation.

5. Outil (2) selon la revendication 4,
**caractérisé en ce que**
le composant proximal (18) présente à son extrémité formant la section de fixation par le biais d'un gradin (18a) un rétrécissement périphérique externe, et le composant distal (17) vient en appui sur la périphérie externe rétrécie, dans lequel une extrémité du composant distal (17) tournée vers le gradin (18a) du composant proximal (18) est écartée du gradin (18a).

6. Outil (2) selon une des revendications 1 à 3,
**caractérisé en ce que**
le composant distal (17) présente à son extrémité formant la section de fixation par le biais d'un gradin (17b) un rétrécissement périphérique externe et le composant proximal (18) présente en correspondance par le biais d'un gradin (18b) un élargissement périphérique interne, dans lequel le composant proximal (18) repose sur la périphérie externe rétrécie du composant distal (17).

7. Outil (2) selon la revendication 5,
**caractérisé en ce que**
une couche isolante, de préférence une isolation en forme de manchon (7), de manière particulièrement préférée une gaine rétrécissable, repose autour du composant proximal (18) et au moins autour d'une partie de la section de fixation,
dans lequel le rétrécissement périphérique externe du composant proximal (18) et/ou une extrémité du composant distal (17) forment une face d'appui pour la couche isolante.

8. Outil (2) selon au moins une des revendications 4 à 7,
**caractérisé en ce que**
le corps d'électrode
- s'étend au moins aussi loin dans le corps de base (5) qu'il atteint jusqu'au gradin (18a) du composant proximal (18), de préférence plus loin que jusqu'au gradin (18a) du composant proximal (18), et/ou
- présente à son extrémité proximale (10a) un diamètre externe épaissi dont la face d'enveloppe repose sur le côté interne du composant proximal (18).

9. Outil (2) selon au moins une des revendications 4 à 8,
**caractérisé en ce que**
l'outil (2) est un outil bipolaire (2) et présente une électrode neutre (8) qui est disposée en forme de manchon coaxialement au composant proximal (18) du corps de base (5).

10. Instrument chirurgical (1) pour l'incision à l'électrocautère avec une poignée (3) et un outil pouvant être connecté de manière amovible à la poignée (3),
**caractérisé en ce que**
l'outil est un outil (2) selon au moins une des revendications 1 à 9.

11. Instrument (1) selon la revendication 10,
**caractérisé en ce que**
la poignée (3) présente à son extrémité proximale un dispositif de raccord (4, 4a), dans lequel le dispositif de raccord (4, 4a) est une connexion électrique de l'électrode (10) à un alimentation électrique et/ou une connexion en forme de tuyau à un dispositif d'aspiration.
